# EUROPEAN PATENT APPLICATION

(11) **EP 2 177 911 A1**
(43) Date of publication of application: **21.04.2010**
(21) Application number: 09012626.9
(22) Date of filing: 06.10.2009
(51) Int. Cl.: G01N 33/96, G01N 33/50

(54) **Hematology controls and methods**

(30) Priority: 08.10.2008 US 103730 P; 05.10.2009 US 573332
(71) Applicant: Streck Inc., La Vista, NE 68128 (US)
(72) Inventor: Hunsley, Bradford A., La Vista, NE 68128 (US); Chen, Lei, Omaha, NE 68135 (US)
(74) Representative: Bawden, Peter Charles

(57) **Abstract**

An integrated hematology control and methods for making the same, including a first cellular component derived from a plurality of processed animal red blood cells other than human blood cells and a second cellular component derived from a plurality of processed animal red blood cells other than human blood cells and a plurality of human blood cells, wherein the control simulates erythroblasts and white blood cells of a human blood sample on an automated blood analyzer.

## Description

### CLAIM OF BENEFIT OF FILING DATE

The present application claims the benefit of the filing date of U.S. Provisional Application Serial No. 61/103,730 (filed October 8, 2008) and U.S. Application Serial No. 12/573,332 (filed October 5, 2009), the entirety of the contents of these applications being hereby expressly incorporated by reference.

### FIELD OF THE INVENTION

This invention relates to simulated blood components and more particularly to simulated blood components for use in controls for automated hematology analyzers.

### BACKGROUND OF THE INVENTION

A popular tool for the analysis of blood is an automated hematology analyzer. In general, such instruments use particle detection technologies to identify the existence of one or more components of whole blood. Such detection technologies may include, for example, particle light scatter detection and particle impedance as measured by DC current, RF frequency, or otherwise. A number of patent documents address controls for use in an automated hematology analyzer such as the COULTER® GEN-S™ Hematology Analyzer or the COULTER® LH series analyzer, available from Beckman Coulter Corp., the ADVIA® 2120 Hematology System from Bayer, the CELL-DYN 4000 from Abbott, and the Sysmex XE-2100™ Automated Hematology Analyzer from Sysmex, or the like for simulating the characteristics of one or more of detectable characteristics of blood cell components. One particular component that has received attention has been nucleated red blood cells, also known as erythroblasts. See, generally, U.S. Patent Nos. 7,135,341; 6,962,817; 6,723,563; 6,653,137; 6,221,668; 6,200,500; 6,187,590; and 5,858,790; all incorporated by reference herein. Another component that is of particular interest in blood screening is white blood cells, specifically testing a sample to obtain cell counts for one or more subpopulations of white blood cells. See, generally, U.S. Patent Nos. 6,509,192; 6,406,915; 6,403,377; 6,362,003; 6,221,668; 5,939,326; and 5,631,165; all incorporated by reference herein.

Notwithstanding the above, there remains a need for improved integrated controls that contain components for simulating both erythroblasts and white blood cells while allowing for clear differentiation between the erythroblast components and white blood cell components, specifically individual white blood cell subpopulation components such as lymphocytes, during analysis. More particularly, there continues to be a need for controls useful in instruments utilizing technology that differentiates blood subpopulations on the basis of one or any combination of cell volume, light scatter and/or conductivity (e.g., such as is employed in the Coulter® VCS technology). There is a further need for integrated controls that can be produced using minimal amounts of human blood components and greater amounts of animal blood components for simulation of human blood components. For some applications, it may be attractive to use animal red blood cells as the starting materials for producing simulated white blood cell components. This has the potential to help manage the handling of human white blood cells and may potentially involve less costly handling. However, it is generally not a matter of simple substitution of one blood cell type for another as the stability of the resulting product and/or the performance of individual instruments is not necessarily predictable. Reproducibility across all desired subpopulations is often difficult to achieve.

The present invention addresses one or more of the above needs by providing improved controls and methods for making the controls.

### SUMMARY OF THE INVENTION

By way of summary, the present invention meets some or all of the above needs by providing in a first aspect an integrated hematology control comprising a simulated erythroblast component and at least one white blood cell subpopulation, (namely a lymphocyte component), wherein only a portion (e.g., a minor portion) of the at least one white blood cell subpopulation (and particularly the lymphocyte component) is derived from human white blood cells and the remaining amount of that subpopulation (e.g., the remainder of the lymphocytes) is derived from one or more animal red blood cells. A desirable approach for the integrated hematology controls herein envisions a simulated erythroblast component in a composition that also includes a simulated lymphocyte component. The simulated erythroblast component may include a plurality of animal red blood cells processed for simulating erythroblasts of a human blood sample on an automated blood analyzer. The lymphocyte component may include a plurality of animal red blood cells processed for simulating lymphocytes of a human blood sample on an automated blood analyzer and a plurality of human white blood cells including a lymphocyte population that will also simulate lymphocytes of a human blood sample on an automated blood analyzer. The resulting erythroblast and lymphocyte components are combined to form a control capable of simulating and differentiating lymphocytes and at least one, two, three, or four other subpopulations.

The various components may be derived from a number of sources. The simulated erythroblast component may include cells derived from avian red blood cells such as chicken red blood cells and/or turkey red blood cells. The simulated erythroblast component may include cells derived from alligator red blood cells. The lymphocyte component may include cells derived from avian red blood cells including chicken red blood cells and turkey red blood cells. The lymphocyte component may include cells derived from human whole blood. The lymphocyte component may include lymphocytes derived from human whole blood. The human whole blood may undergo certain processing steps including a red blood cell lysing step. The animal red blood cells included in the lymphocyte component may make up at least 50% of the lymphocytes in the lymphocyte component. The resulting control may exhibit a histogram that includes a valley between peaks associated with the erythroblast population and the lymphocyte population. The resulting control may also exhibit a white blood cell five part differential.

In another aspect, the present invention contemplates a method for making an integrated hematology control, comprising providing a component that simulates erythroblasts, providing a component that simulates lymphocytes and providing a component that simulates at least two subpopulations of white blood cells. The component that simulates erythroblasts may be produced by providing a first portion of one or more animal red blood cells having a first volume, shrinking the first portion of one or more animal red blood cells to a volume at least 10% smaller than the first volume, contacting the first portion of one or more animal red blood cells with a surfactant, and fixing the first portion of one or more animal red blood cells. The component that simulates lymphocytes may be produced by providing a second portion of one or more animal red blood cells, contacting the second portion of one or more animal red blood cells with a surfactant and fixing the second portion of one or more animal red blood cells. The component that simulates at least two subpopulations of white blood cells may be produced by providing a sample of whole human blood, lysing the red blood cells from the sample of whole human blood so that white blood cells remain, stabilizing the white blood cells and removing a portion of lymphocytes from the stabilized white blood cells. The removed portion of lymphocytes may then be replaced with the component that simulates lymphocytes. The component that simulates erythroblasts, and the component that simulates at least two subpopulations of white blood cells may then be combined to form the integrated control.

In another aspect, the present invention contemplates an integrated hematology control, comprising a plurality of chicken red blood cells processed for simulating an erythroblast population of a human blood sample on an automated blood analyzer, and a sample of human whole blood processed to remove the red blood cells. A portion of the remaining white blood cells may include a lymphocyte population and a portion of the lymphocyte population is removed and replaced with a plurality of turkey red blood cells processed for simulating lymphocytes of a human blood sample on an automated blood analyzer such that less that 50% of the resulting lymphocytes are derived from human lymphocytes. The control further exhibits a white blood cell five part differential and exhibits a histogram that includes a valley between peaks associated with the erythroblast population and the lymphocyte population.

By the practice of the teachings herein, it can be seen how to achieve a stable hematology control (e.g., consistent and reproducible results for at least 10 days from manufacture date, and more specifically at least 1 month, 2 months or even 6 months from manufacture date) that simulates at least one white blood cell population (e.g., lymphocytes), and more preferably two, three, four, or even five subpopulations. By selection of a combination of animal red blood cells it also becomes possible to include a simulated erythroblast component in combination with the simulated white blood cell subpopulations as an integrated control by which a readout from a hematology analyzer corresponds with the lymphocyte subpopulation. Advantageously, the use of animal red blood cells for at least part of the white blood cell component (e.g., at least part of the lymphocyte component) permits for cost effective manufacturing and relative ease in handling.

### BRIEF DESCRIPTION OF THE DRAWINGS

FIG. 1 compares an example scattergram result of an illustrative prior art control and the scattergram results of an illustrative control of the present invention.
FIG. 2 compares an example histogram result of an illustrative prior art control and histogram results of an illustrative control of the present invention.

### DETAILED DESCRIPTION

The present invention is predicated upon a unique combination of cellular components which are able to simulate populations of both erythroblasts and white blood cells when passed through an automated hematology analyzer. The combination makes the overall composition attractive for use as an "integrated" control, i.e., a control that contains more than one type of cellular component and can effectively reduce the need for multiple stand-alone controls. Thus, in one aspect, the invention is directed to an integrated control; that is, a plurality of blood cells processed for simulating an erythroblast population and a plurality of blood cells processed for simulating a white blood cell population wherein the control exhibits at least a white blood cell three part differential or possibly even a five part differential. That is, the single integrated control is capable of being processed on a hematology analyzer to produce an output that resembles a whole blood sample that includes erythroblasts and at least three if not all five subpopulations of white blood cells. Of course, it will be appreciated that the compositions herein can be modified in accordance with art-disclosed teachings to arrive at a control that functions to simulate any combination of one or more cell populations normally encountered in whole blood (e.g., human whole blood). For instance, one possible approach contemplates a control that simulates detectable characteristics of an erythroblast population and a white blood cell population (and corresponding subpopulations) plus one or a combination of platelets, reticulated platelets, reticulocytes, immature granulocytes (e.g., per U.S. Patent No. 7,109,036, incorporated by reference), or any combination thereof. Thus, without limitation, the controls of the present invention may be employed in combination (e.g., as a mixture, a kit comprising discrete individual controls, or both) with a control as disclosed in U.S. Patent Nos. 6,723,563; 6,653,137; 6,221,668; or 6,200,500, all incorporated by reference. It will be appreciated that one or more of the simulated blood cell components in the controls herein may include a synthetic particle (e.g., a synthetic polymer, such as polystyrene), as discussed in U.S. Patent No. 6,962,817, incorporated by reference.

The control compositions of the present invention provide components which provide accurate counts of both erythroblasts and white blood cells in addition to other components of whole blood. More particularly, the controls herein provide a count corresponding with predicted values for white blood cells, including counts for each of the five subpopulations of white blood cells.

In general, the control compositions herein exhibit long term stability, and particularly stability that substantially exceeds the stability of a fresh whole blood (e.g., human blood) sample containing fresh erythroblasts and white blood cells (e.g., human erythroblasts and human white blood cells). For example, the controls, their respective cellular components, or both, have a useful life of at least 10 days from manufacture date, and more specifically at least 1 month, 2 months or even 6 months from manufacture date.

It is contemplated that the controls of the present invention comprise a plurality of subgroups of human or animal red or white blood cells that have each been processed independent of the other according to individual processing parameters, before being combined in a common suspension liquid, such as to form the resulting control composition. For instance, in one approach the suspended particles include at least two, at least three, at least four, or even at least five individually processed subgroups of animal or human blood cells. The processing of animal cells to simulate erythroblasts and white blood cells has been addressed throughout the art. See e.g., U.S. Patent Nos. 7,135,341; 6,723,563; 6,187,590; and 5,858,790, all incorporated by reference. Examples of suitable blood cell sources include, without limitation, humans, mammals, birds, reptiles, and fish. Specific cell sources are alligators, turkeys, geese, chickens, sharks, cows, pigs, goats, salmon, trout, or another source of a blood cell.

According to the teachings herein, when a plurality of subgroups of animal or human blood cells are used, the starting blood cells may be cells from the same type of source (e.g., a human or a specific type of animal), or from different types of sources. Two or more of the cells may be prepared according to a different set of process conditions. For example, each of two, three, four, or five subgroups may be cells from a reptile, or they may be cells from a reptile and a bird, a bird and fish, a mammal, bird, fish and reptile. Of course it is also possible to employ human blood cells that have been processed for realizing long term stability. It is possible herein that an erythroblast or white blood cell population may be simulated based upon processing of a blood cell. One specific preferred embodiment for providing an integrated control includes a plurality of different types of blood cells, (e.g., a chicken cell processed according to a first set of conditions, and a turkey cell processed according to a second set of conditions different from the first set of conditions, and a processed human white blood cell; one or more processed shark cells and one or more processed alligator cell; a processed turkey cell, a first processed alligator cell, and a second processed alligator cell that is processed in a manner different than the first processed alligator cell; any combination of the foregoing, or otherwise). One unique aspect of the teachings herein is that two or more different starting cells are used to simulate a single subpopulation.
As mentioned above, a portion of the white blood cell component may be derived from animal red blood cells. In one preferred embodiment, a portion of the white blood cell component derived from human white blood cells is replaced with one or more white blood cell analogs derived from animal red blood cells. In a more preferred embodiment, the one or more white blood cell analogs derived from animal red blood cells are processed to simulate lymphocytes.

For example, at least 50% of the lymphocytes in the resulting white blood cell component may be lymphocytes derived from the animal red blood cells. In a preferred embodiment, at least 70% of the lymphocytes in the resulting white blood cell component may be lymphocytes derived from the animal red blood cells. In another preferred embodiment, at least 90% of the lymphocytes in the resulting white blood cell component may be lymphocytes derived from the animal red blood cells.

In general, the starting cells are received from the blood source and are washed and separated from the remainder of the blood components. For example, the cell source blood is received as whole blood that has been contacted with a first suitable suspension solution, e.g., an Alsever's solution, Hank's solution or otherwise. The cells are typically washed with a second suitable solution (e.g., an isotonic wash solution that is the same as or different from the first suitable suspension solution), before, during, and/or after (or any combination thereof) a separation step, such as a step of separation by a separation technique such as filtration, centrifugation, sedimentation, or any combination thereof. In general, a single blood cell population (e.g., animal red blood cells, human white blood cells or another blood cell population) is separated from the remainder of the blood. The separated cells may then be re-suspended in a third suitable suspension solution (the same as or different from the first and/or second suspension solutions). It is possible that the suspension solution, the isotonic wash solution or both may include distilled and/or de-ionized water; and one or more of a fungicide of up to about 5 parts; an antimicrobial of up to about 5 parts; a surfactant ranging from about 5 parts to about 20 parts; a buffer ranging from about 5 parts to about 30 parts, a metal chelating agent ranging from about 25 parts to 50 parts; a cell nutrient of up to about 5 parts; and an agent for maintaining tonicity in about 15 parts to about 35 parts; or any combination thereof. By way of example, without limitation, the liquid suspension medium may include or consist essentially of a composition selected from one or any combination of the compositions of Disodium EDTA, Magnesium Gluconate, Na₂HPO₄, PEG 20,000, Inosine, Glucose, Na Fluoride, NaOH, BSA, Sulfasalazine, Methyl Paraben, Neomycin Sulfate or Chloramphenicol. Examples of suitable media may also be gleaned from one or more of U.S. Patent Nos. 7,135,341; 6,723,563; 6,653,137; 6,221,668; 6,200,500; 6,187,590; and 5,858,790; all incorporated by reference herein.

The isotonic solution may also contain other ingredients as described in U.S. Patent Nos. 5,858,790 or 6,187,590 (incorporated by reference herein). Optionally, though certain of the above may already perform such functions, the solution may also include one or more agents that function as a hemolysis inhibitor, an aggregating agent, cell size, shape or volume stabilizer, metabolite, protein source, an agent for properly positioning the white blood cell subpopulation (e.g., a lipoprotein), an antioxidant, a debris reducer or a mixture thereof.

The cells may be processed for preserving their size, content or both; for preserving one or more antigens on a cell surface, or any combination During the cell processing step, typically one or more techniques are performed for: increasing cell volume; decreasing cell volume; removing hemoglobin; removing cytoplasm; cross-linking a membrane; cross-linking a cell content; denaturing cell content; denaturing a membrane; removing a membrane; repairing a membrane; introducing a substance within a membrane; or any combination thereof. For example, one approach is to employ a process including or consisting essentially of steps of increasing cell volume; and cross-linking or otherwise denaturing a cell membrane.

Any of a number of art-disclosed techniques may be employed, such as those described in U.S. Patent Nos. 7,135,341; 6,723,563; 6,653,137; 6,221,668; 6,200,500; 6,187,590; and 5,858,790; all incorporated by reference herein. Among the techniques, for example, are thermal processing, chemical processing, or both. Chemica! processing involves processing with a chemical for achieving a chemical reaction on or within a cell, a physical modification of the cell or its contents, or any combination thereof. Multiple techniques may be performed simultaneously or sequentially upon a particular cell. Preferably the solution will include at least one or more, more preferably two or more, still more preferably three or more, still more preferably four or more and still even more preferably all of the following ingredients: a fungicide; an antimicrobial; a surfactant; a buffer; a metal chelating agent; a cell nutrient; or an agent for maintaining tonicity.

It is possible that cells may be subjected to one or more processing steps, each for realizing a separate result. For example, a cell may be swollen or shrunk, and then fixed. A cell may be swollen or shrunk, and hemoglobin removed, and then fixed. A cell may be swollen or shrunk, RNA introduced to the cell and then fixed. A cell may have a membrane removed and the nucleus fixed. A cell may simply be fixed. A cell may be swollen or shrunk and then remain unfixed. A cell may be contacted with a lipoprotein (e.g., as taught in U.S. Patent Nos. 5,270,208; 5,262,327; and 6,723,563, incorporated by reference). It will be appreciated that even though the teachings herein are specifically exemplified by reference to particular integrated controls, they are not intended to be so limited. Various of the techniques herein may be employed for preparing controls for simulating other blood cell components.

In the past, the use of hypotonic solutions to swell cells has been employed. See, U.S. Patent No. 5,320,964. It is also contemplated, for instance, that cells from a blood source can be contacted with a hypertonic solution for causing a change in cell size. A blood cell thus can be processed by contacting with a hypertonic or hypotonic solution of sufficient concentration and at a suitable temperature for expanding or reducing the cell size to approximate the size of one or more components of whole blood. Examples of hypertonic solutions could include, without limitation, solutions of one or more of a salt or a sugar. By way of more specific examples, the solution may be a metal halide solution (e.g., one or more chlorides, bromides, or iodides of one or more of sodium, magnesium, calcium, or potassium), any combination thereof, or the like; polyols (e.g., ethylene glycol, propylene glycol, glycerol), alkyl sulfoxides (e.g., dimethyl sulfoxide), alkyl formamides (e.g., dimethylformamide), alkyl acetamides (e.g., dimethylacetamide), a urea, or any combination thereof.

When employed for changing cell size, the process herein can be employed for altering the volume relative to the original cell volume by as much as 70% or greater. Ordinarily however, volume change will be less than 50%, or even less than 30% (e.g., as low as about 10% or even 20%).

Without limitation, examples of various media are also provided in the Following Table 1 and Table 2, illustrating examples of compositional ranges for the solutions useful herein. The ultimate concentrations may be varied as desired to take into account the particular materials and processing conditions selected by the user. For example, one approach may be to select a concentration (in mg%) that falls toward the central portion of the stated ranges of Table 1, and with reasonable experimentation select the ultimate optimal concentration for the specific application. In addition to the following it is possible that a phosphate buffered solution (which may itself include one or more other ingredients, such as a surfactant (e.g., a suitable concentration of a cationic, anionic, or even a nonionic surfactant, such as 1% w/v Triton X). Hank's solution, or a composition including the same may also be employed.

**Table 1**

| TABLE 1 Chemical | A (mg%) | B (mg%) | C (mg%) | D (mg%) | E (mg%) | F (mg%) |
|---|---|---|---|---|---|---|
| EDTA (NA2) | 1500-1820 | 1100-1250 | 1100-1250 | 600-800 | 600-800 | 90-110 |
| Mg Gluconate | 880-980 | 600-700 | 600-700 | 360-420 | 360-420 | - |
| NA2HP04 | 600-680 | 400-500 | 400-500 | 240-300 | 240-300 | 60-80 |
| PEG-20k | 550-850 | 550-850 | 550-850 | 550-850 | 550-850 | 850-1100 |
| Inosine | 20-30 | 20-30 | 20-30 | 10-30 | 80-120 | - |
| Glucose | - | 1000 | 800-1200 | 500-700 | 500-700 | - |
| NaF | - | 3-7 | 3-7 | - | - | - |
| NaOH | 180-225 | 120-160 | 120-160 | 75-85 | 75-95 | - |
| BSA | - | - | 2000 | - | - | - |
| Sulfasalazine | - | - | 10 | - | - | - |
| CaCl2 | - | - | - | - | - | - |
| KH2PO4 | - | - | - | - | - | 1-5 |
| NaCl | - | - | - | - | - | 600-900 |
| MgSO4 * 7H2O | - | - | - | - | - | - |
| KCI | - | - | - | - | - | - |
| NaHCO3 | - | - | - | - | - | - |
| Ciprofloxacin | - | - | - | - | - | - |
| Methylparaben | 30-50 | 30-50 | 30-50 | 30-50 | 30-50 | - |
| Neomycin SO4 | 30-50 | 30-50 | 30-50 | 30-50 | 30-50 | - |
| Chloramphenicol | 12-20 | 12-20 | 12-20 | 12-20 | 12-20 | 12-20 |
| **PH** | **7.10 ± 0.02** | **7.10 ± 0.02** | **7.10 ± 0.02** | **7.00 ± 0.02** | **7.10 ± 0.02** | **7.20 ± 0.02** |
| Osmolarity | **315 ± 10** | **295 ± 10** | **295 ± 10** | **300 ± 5** | **300 ± 10** | **280 ±10** |

**Table 2**

| Chemical | | | A mg% | mM | B mg% | mM | C mg% | mM | D mg% | mM | E mg% | mM | F mg% | mM | G mg% | mM |
|---|---|---|---|---|---|---|---|---|---|---|---|---|---|---|---|---|
| | | EDTA (NA2) | 1675 | 45 | 1173 | 31.5 | 1173 | 31.5 | 704 | 18.9 | 704 | 18.9 | 100 | 2.7 | - | - |
| | | Mg Gluconate | 933 | 22.5 | 653 | 15.7 | 653 | 15.7 | 392 | 9.45 | 392 | 9.45 | - | - | - | - |
| | | NA2HP04 | 639 | 45 | 447 | 31.5 | 447 | 31.5 | 268 | 18.9 | 268 | 18.9 | 73 | 5.1 | 4.9 | 0.34 |
| | | PEG-20k | 700 | 0.35 | 700 | 0.35 | 700 | 0.35 | 700 | 0.35 | 700 | 0.35 | 1000 | 0.5 | - | - |
| | | Inosine | 25 | 0.93 | 25 | 0.93 | 25 | 0.93 | 25 | 0.93 | 100 | 3.7 | - | - | - | - |
| | | Glucose | - | - | 1000 | 55.5 | 1000 | 55.5 | 600 | 33.3 | 600 | 33.3 | - | - | 100 | 5.5 |
| | | NaF | - | - | 5 | 1.2 | 5 | 1.2 | - | - | - | - | - | - | - | - |
| | | NaOH | 204 | 51 | 140 | 35 | 140 | 35 | 80 | 20 | 85 | 21.25 | - | - | - | - |
| | | BSA | - | - | - | - | 2000 | - | - | - | - | - | - | - | - | - |
| | | Sulfasalazine | - | - | - | - | 10 | 0.25 | - | - | - | - | - | - | - | - |
| | | CaCl2 | - | - | - | - | - | - | - | - | - | - | - | - | 14 | 1.3 |
| | | KH2PO4 | - | - | - | - | - | - | - | - | - | - | 2 | 0.15 | 6 | 0.44 |
| | | NaCl | - | - | - | - | - | - | - | - | - | - | 830 | 142 | 800 | 136.9 |
| | | MgSO4 * 7H2O | - | - | - | - | - | - | - | - | - | - | - | - | 20.1 | 0.82 |
| | | KCI | - | - | - | - | - | - | - | - | - | - | - | - | 40 | 5.4 |
| | | NaHCO3 | - | - | - | - | - | - | - | - | - | - | - | - | 35 | 4.2 |
| | | Ciprofloxacin | - | - | - | - | - | - | - | - | - | - | - | - | 2 | - |
| | | Methylparaben | 40 | 2.6 | 40 | 2.6 | 40 | 2.6 | 40 | 2.6 | 40 | 2.6 | - | - | 40 | 2.6 |
| | | Neomycin SO4 | 40 | 0.44 | 40 | 0.44 | 40 | 0.44 | 40 | 0.44 | 40 | 0.44 | - | - | 40 | 0.44 |
| | | Chloramphenicol | 15 | 0.46 | 15 | 0.46 | 15 | 0.46 | 15 | 0.46 | 15 | 0.46 | 15 | 0.46 | 15 | 0.46 |
| | | **PH** | **7.10 ± 0.02** | | **7.10 ± 0.02** | | **7.10 ± 0.02** | | **7.00 ±0.02** | | **7.10 ± 0.02** | | **7.20 ± 0.02** | | **7.40 ± 0.20** | |
| | | **Osmolarity** | **315 ± 10** | | **295 ± 10** | | **295 ± 10** | | **300 ± 5** | | **300 ± 10** | | **280 ±10** | | **280 ± 10** | |

In the context of the integrated controls herein, the control will function to simulate human erythroblasts and human white blood cells, including multiple subpopulations (e.g., three, four, or five) of human white blood cells for allowing differentiation of the individual subpopulations. Thus, methods herein contemplate detecting and counting of simulated erythroblasts and detecting and counting of simulated white blood cells, with white blood cell subpopulation differentiation.

What follows is a summary of certain particular approaches for preparing blood cell components (e.g., an erythroblast component and a white blood cell component) for use in a control in accordance with the present invention. One process includes the steps of providing a source of blood cells and separating and removing unwanted components. The remaining components are processed to cause them to simulate one or more components of whole blood; and the components are optionally processed (e.g., fixed) for preserving long term stability. Resulting components may then be re-suspended in a desirable suspension medium.

### White Blood Cell Component

In the present embodiment, the component of the control that is to simulate the characteristics of white blood cells in whole blood is prepared from a biological material. More specifically, the material is a cellular biological material, and preferably the material includes human white blood cells and animal red blood cells (although any suitable blood cells may be employed). In a preferred embodiment of the present invention, the white blood cell component includes materials for replicating the relevant measurable characteristics for some or all of each of the five white blood cell subpopulations, namely, lymphocytes, monocytes, neutrophils, eosinophils and basophils.

The white blood cell component of the hematology control composition may comprise a blood cell or analog thereof, wherein subpopulations of the white blood cell component (e.g., at least two, or even at least three subpopulation) are derived from human white blood cells and additional portions of the white blood cell component are derived from animal red blood cells. The human white blood cells and the animal red blood cells may be processed separately and later combined. A portion of the white blood cell component derived from human blood may be replaced by the white blood cell component derived from the animal red blood cells. A source of human whole blood may be processed to stabilize the white blood cell subpopulations (and, of course, to lyse and/or remove any undesired other components). Before or after stabilizing, at least a portion of (e.g., more than one half of) the lymphocyte portion derived from the human whole blood is removed and replaced with lymphocytes derived from the animal red blood cells (e.g., derived from an avian red blood cell).

The human white blood cells are provided in packs (e.g., 2 to 3 packs per bottle). The cells are treated by a series of steps for selectively lysing any undesired blood cells present in the material as provided; for washing the cells; and for fixing or otherwise stabilizing the cells. Selective lysing may be accomplished in any suitable manner, for instance, by contacting the undesired blood cells with a lysing agent. Any suitable lysing agent may be employed. Buffered halides, such as ammonium chloride and Tris Base (e.g., about 7.5 g ammonium chloride and 2 g Tris per liter), illustrates one suitable class of lysing agents, where the undesired cells include red blood cells. Lysing may be accomplished through a series of consecutive washing steps with the lysing agent. Optionally, before the lysing, the cells are subjected to a preliminary fixing step, such as by contacting them with a suitable fixing agent, heating them or both. For instance, the cells are contacted with a buffered antimicrobial saline solution including a suitable amount of a fixative (e.g., about 0.11% formaldehyde). In one preferred embodiment, a hypotonic solution may be used to cause the cells to swell. See, U.S. Patent No. 5,320,964.

The white blood cells may also be fixed for stabilization. The fixative may include but is not limited to an aldehyde, oxazolidine, alcohol, cyclic urea, or the like. Examples of such fixatives include, without limitation, formaldehyde, glutaraldehyde, diazolidinyl urea (DU), imidazolidinyl urea (IDU), dimethylol urea, dimethylol-5,5- dimethylhydantoin, 2-bromo-2-nitropropane-1,3-diol; quaternary adamantine, hydroxyl-methyl-1-aza-3,7-dioxabicyclo (3.3.0)octane, sodium hydroxymethyl glycinate, and mixtures thereof, or the like. Other fixatives may be used such as those disclosed in U.S. Patent Nos. 5,196,182; 5,262,327; 5,460,797; 5,811,099; 5,849,517; 6,221,668; 5,529,933; 6,187,590 (incorporated by reference herein). The appropriate fixative reagent is selected based upon the cell attribute to be evaluated by a hematology analyzer. In a preferred embodiment, the present invention utilizes a fixation process to generate the control composition. In one particular embodiment of the present invention, the blood cell fixation begins by contacting the washed blood cells with a fixative reagent. Prior to contacting the blood cells, the fixative may be diluted with water, isotonic solution, hypertonic solution, hypotonic solution or any mixture thereof. The ratio of fixative to diluent ranges from 1:1 to 1:300. More preferably, the ratio of fixative to diluent ranges from 1:1 to 1:100. Most preferably, the ratio of fixative to diluent ranges from 1:3 to 1:50.

The animal red blood cell source for deriving the white blood cell analog component is typically received suspended to include a plurality of cells in a liquid medium, and may include one or more ingredients such as an anticoagulant. Accordingly, it may be desirable to separate the cells from the as-provided liquid medium and to wash the cells. One or more separation steps may be employed, such as centrifugation, filtration or both. Typically, washing will employ contacting the cells with a suitable solution, preferably a buffered solution, and most preferably a suitable isotonic wash solution.

During processing of the animal red blood cells, it may be possible to avoid any intermediate processing step following the washing step, and to proceed immediately to a stabilization step, such as a fixing step to preserve the structure of the cell for achieving long-term (e.g., at least about 30 days, more preferably at least about 45 days, or at least about 60 days, and possibly even 90 days or longer) storage. In general, the fixing step will include a step of contacting the cell with a suitable concentration of a fixative, a step of heating, or a combination thereof, for a predetermined amount of time. The amount of time required for fixation varies depending on cell concentration, temperature and the strength of the fixative reagent. Various approaches to fixing are illustrated throughout the literature, including in U.S. Patent Nos. 6,723,563; 6,653,137; 6,221,668; 6,200,500; 5,320,964; all incorporated by reference.

The fixative may include but is not limited to an aldehyde, oxazolidine, alcohol, cyclic urea, or the like. Examples of such fixatives include, without limitation, formaldehyde, glutaraldehyde, diazolidinyl urea (DU), imidazolidinyl urea (IDU), dimethylol urea, dimethylol-5,5- dimethylhydantoin, 2-bromo-2-nitropropane-1,3-diol; quaternary adamantine, hydroxylmethyl-1-aza-3,7-dioxabicyclo (3.3.0)octane, sodium hydroxymethyl glycinate, and mixtures thereof, or the like. Other fixatives may be used such as those disclosed in U.S. Patent Nos. 5,196,182; 5,262,327; 5,460,797; 5,811,099; 5,849,517; 6,221,668; 5,529,933; 6,187,590 (incorporated by reference herein). The appropriate fixative reagent is selected based upon the cell attribute to be evaluated by a hematology analyzer. In a preferred embodiment, the present invention utilizes a fixation process to generate the control composition. In one particular embodiment of the present invention, the blood cell fixation begins by contacting the washed blood cells with a fixative reagent.

In one preferred embodiment, the portion of the white blood cell component derived from human white blood cells may be further treated to reduce the lymphocyte count within the analog. In one preferred embodiment, the cells are washed and centrifuged (and supernatant removed) under refrigerated conditions multiple times until the lymphocyte percentage is reduced. In another preferred embodiment, the animal red blood cells processed for simulating lymphocytes are added to the white blood cell component to replace the removed lymphocytes.

### Erythroblast Component

The source blood for deriving the erythroblast component is typically received suspended to include a plurality of cells in a liquid medium, and may include one or more ingredients such as an anticoagulant. Accordingly, it may be desirable to separate the cells from the as-provided liquid medium and to wash the cells. One or more separation steps may be employed, such as centrifugation, filtration or both. Typically, washing will employ contacting the cells with a suitable solution, preferably a buffered solution, and most preferably a suitable isotonic wash solution.

The wash solution, which generally will be substantially isotonic, may include any of a number of ingredients in a deionized and/or distilled water base. For example, the wash solution may include at least one or more, more preferably two or more, still more preferably three or more, still more preferably four or more and still even more preferably all of the following ingredients: a fungicide; an antimicrobial; a surfactant; a buffer; a metal chelating agent; a cell nutrient; or an agent for maintaining tonicity. For example, in one particular embodiment of the present invention, the relative amounts of the above ingredients may be as follows: a fungicide of up to about 5 parts; an antimicrobial of up to about 5 parts; a surfactant ranging from about 5 parts to about 20 parts; a buffer ranging from about 5 parts to about 30 parts, a metal chelating agent ranging from about 25 parts to 50 parts; a cell nutrient of up to about 5 parts; and an agent for maintaining tonicity in about 15 parts to about 35 parts.

The wash solution may also contain other ingredients as described in U.S. Patent Nos. 5,858,790 or 6,187,590 (incorporated by reference herein). The isotonic wash solution may also include ingredients that act as a hemolysis inhibitor, an aggregating agent, a cell stabilizer, an antioxidant, or a mixture thereof. By way of example, one possible wash solution may include about 40 mg % Methyl Paraben, about 300 mg % Polyethylene Glycol - (molecular weight about 20,000); about 1675 mg % Ethylenediaminetetraacetic Acid; about 933 mg % Magnesium Gluconate; about 639 mg % Sodium Phosphate Dibasic anhydrous, about 25 mg % Adenosine, about 25 mg % inosine; about 40 mg % Neomycin Sulfate; and about 15 mg % Chloramphenicol.

The blood cells may then be fixed. The fixatives may include but are not limited to the fixatives discussed above, such as an aldehyde, oxazolidine, alcohol, cyclic urea, or the like.

The fixation may take place at room temperature or at a temperature below room temperature or at a temperature above room temperature. If the fixation takes place below room temperature, the cooling is set to at least about 15°C. More preferably the fixation will take place at a temperature in the range of 15°C to 0°C. Even more preferably, the fixation will take place at a temperature in the range of 10°C to 5°C. Most preferably the fixation will take place at 6°C. If the fixation takes place above room temperature, the heating is set to at least about 30°C. More preferably the fixation will take place at a temperature in the range of 30°C to 50°C. Even more preferably, the fixation will take place at a temperature in the range of 35°C to 40°C. Most preferably the fixation will take place at 40°C.

In a preferred embodiment of the present invention, the fixed blood cells are washed out of the fixative reagent and re-suspended into a suitable suspension medium. The cells can be washed out of the fixative reagent with a buffered isotonic solution.
In one preferred embodiment, the animal red blood cells can be processed by contacting with a hypotonic solution. In another preferred embodiment, the cells can be contacted with an alcohol in an effort to reduce cell size.

In one preferred embodiment, after contact with the hypotonic solution, the cells are resuspended in a diluent. In one preferred embodiment, the cells are resuspended, for instance, in a phosphate buffered solution containing polyethylene glycol 20,000 (PEG), ethylenediamine tetraacetic acid (EDTA) and magnesium gluconate with 2% bovine serum albumin. In another preferred embodiment, the resulting cells are then combined with the cells processed to simulate white blood cells to form an integrated control.

Though disclosed herein in the context of a control composition, in one aspect, the present invention pertains to a blood cell control, which includes or consists essentially of a first component including a plurality of processed animal red blood cells other than human blood cells; a second cellular component including a plurality of processed animal red blood cells other than human blood cells and a plurality of processed human blood cells; wherein at least a portion of the control functions to simulate erythroblasts of a human blood sample on an automated blood analyzer and a portion of the control functions to simulate white blood cells of a human blood sample on an automated blood analyzer.

Cells from the first cellular component of the control will function to simulate erythroblasts of a human blood sample on an automated blood analyzer that analyzes blood cells using impedance, optical measurements or both. Further, the second cellular components are detected by the analyzer as white blood cells. It is further possible that, upon analysis of the cells by the analyzer, the cells of the cellular components provide a white blood cell differentiation (e.g., a three subpopulation differential, a five subpopulation differential or both) within ranges consistent with normal human whole blood (e.g., within the ranges of about Lymphocytes - 20 to 45%; Monocytes - 2 to 10%; Neutrophils - 40 to 75%; Eosinophils - 1 to 6%; and Basophils up to 1 %).

The invention also can be regarded as a hematology control which includes a first part that includes one or more cellular components processed for simulating erythroblasts of a human blood sample on an automated blood analyzer; and a second part that includes at least one cellular component processed for simulating white blood cells of a human blood sample on an automated blood analyzer; wherein upon passing through an automated hematology analyzer the cellular components of the first part are detected by the analyzer as an erythroblast, and the cellular components of the second part is detected as a white blood cell. In such instance, any of the above described sources of blood cells may be employed. However, desirably, the cellular components include avian red blood cells. As with the above, the cellular components of the second part, upon analysis by the analyzer may provide a white blood cell differentiation (e.g., a three subpopulation differential, a five subpopulation differential or both) within ranges consistent with normal human whole blood (e.g., within the ranges of about Lymphocytes - 20 to 45%; Monocytes - 2 to 10%; Neutrophils - 40 to 75%; Eosinophils - 1 to 6%; and Basophils up to 1%).

As will be seen, in certain respects, the invention also pertains to a method of making a control for resembling an erythroblast population and a white blood cell population, comprising the steps of admixing in an aqueous diluent: a first cellular component (as described above) including a plurality of processed animal red blood cells other than human blood cells; and a second cellular component (as described above) including a plurality of processed animal red blood cells other than human blood cells and a plurality of processed human white blood cells; wherein the resulting control simulates erythroblasts and white blood cells of a human blood sample on an automated blood analyzer that analyzes blood cells using impedance, optical measurements or both.

The control composition is prepared and analyzed by the same standard method as test samples which may be tested in batch quantities by the use of a suitable cassette having apertures for receiving test vials. After preparation, the control composition and test samples are analyzed by detecting the presence of or counting the population number of each subject component type with a multi-parameter automated hematology instrument, which will preferably yield a visual display of the data. In one embodiment the control of the present invention is provided in combination with a peripheral device, such as a device for tracking samples and associating them with particular data, e.g., a bar-code scanner system, an RFID system or otherwise. The control may also be provided in combination with a slide preparation kit, stain or dye-resistant labels, lytic reagents (e.g., containing a quaternary ammonium salt), blood diluents, or other like components used in a clinical laboratory setting.

The automated test instrument may employ technology that analyzes cell samples in view of simultaneous volume conductivity and light scatter measurements, or solely by light scatter. Ordinarily, a starting sample is employed in combination with suitable reagents (which may comprise a component of a kit) and physical agitation for lysing and cell measuring by way of flow cytometry.

Examples of the various analysis techniques that might be employed will be apparent by familiarity with the above identified commercially available instruments, as well as by reference to art-disclosed techniques discussed in U.S. Pat. No. 6,060,322 (discussing "mixing a blood cell sample containing reticulated cells with a reagent composition comprising a metachromatic dye and a sphering agent to form a suspension of cells"; U.S. Pat. No. 6,232,125 (stating that "Light scattering characteristics of the leukocytes are determined within five different angular ranges, all being lower than 40 degrees"); U.S. Pat. No. 6,228,652 (discussing use of "single transducer for simultaneously measuring the DC volume, RF conductivity, light scattering and fluorescence characteristics of blood cells passing through a cell-interrogation zone"); U.S. Pat. No. 5,917,584 (discussing "differentiation and enumeration of nucleated red blood cells without using fluorescence"); U.S. Pat. No. 5,874,311 (discussing "measuring low angle light scatter signal detected in less than 10° to differentiate reticulocytes from other cell types") U.S. Pat. No. 5,874,310 (discusses "exposing a blood cell sample to a reagent system to lyse mature red blood cells and subsequently analyzing nucleated red blood cells in a flow cell by optical analysis" and the use of "two angles of light scatter signals" such as "low angle light scatter signals detected in less than 10°"). Other techniques are also discussed in U.S. Pat. Nos. 5,858,790 and 6,187,590. Of course, by no means is the mode of sample testing limited to the above.

As mentioned, other principles may be used. One preferred approach is to employ the controls of the present invention in a process (e.g., by passing the control through a suitable instrument) by which erythroblasts are differentiated from other cell types using axial light loss and low angle light scatter measurements, (e.g., from about 1° to 9°, and more specifically from about 3° to 7°), axial light loss and DC impedance measurements, or any combination thereof. Preferably the analyzer is also adapted for differentiating white blood cells and for analyzing a blood cell sample by light scatter (e.g., by measuring low angle light scatter signals), and more preferably by two angles of light scatter measurement, which may include a medium angle light scatter signal and a right-angle light scatter. Preferably both signals (or only one if a single angle scatter is used) are less than about 10° (e.g., from about 1 to about 7°, or for some multi-angle scatter measurements, one light scatter angle may be in the range of about 0 to about 4° and the other light scatter angle is in the range of about 3 to about 7°) to differentiate erythroblasts and white blood cells or lymphocytes from other cell types. The control is passed through the hematology analyzer at a suitable temperature (e.g., at a temperature in the range of about 18 to about 28°C., though higher or lower temperatures are also possible). It is possible that the analyzer detects cells by measuring axial light loss signals and DC impedance. The analyzer may detect cells by measuring low angle light scatter signals in combination with measuring axial light loss signals and DC impedance. In one embodiment, the control is passed though the instrument only a single time, in order to obtain a satisfactory result. In another embodiment, the control is repeatedly passed though the instrument to assure test integrity.

The results of the analysis, which will resemble that of whole blood, may then be analyzed and reported. For example, the respective population counts obtained from the analysis are compared either to known reference value for each component type in the control composition, or by comparison of the population counts for each component types in the test sample with the corresponding values of components in the control composition. Data relating to the measurement of components in control composition and test samples is collected, monitored, stored, compared and analyzed by electronic means, such as part of a system including a computer programmed with appropriate software and containing appropriate data file structure, and preferably coupled with one or more devices for outputting or storing the data (e.g., a monitor, a printer, an electronic data storage medium or the like).

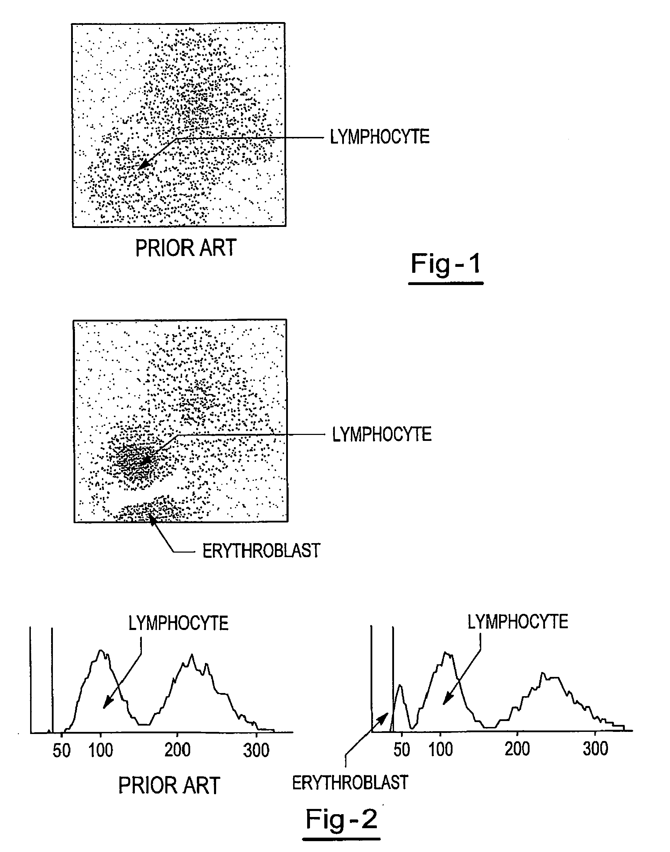

The data obtained from the analysis may be displayed in scattergram or histogram form as depicted in FIG. 1 and FIG. 2. The scattergram of hematology analysis of the prior art does not display distinct scatter results between the lymphocytes and erythroblasts of an integrated sample. However the present invention displays a scattergram with a distinct separation between the scatter results of the lymphocytes and those of the erythroblasts, as depicted in FIG. 1. The histograms shown in FIG. 2 display similar results, showing a distinct valley between the lymphocyte count and erythroblast count of the present invention as compared with the histogram of the prior art where no valley between the lymphocyte and erythroblast values is visible.

The skilled artisan will appreciate that a number of the steps and ingredients have been disclosed by way of example, but that any of a number of alternative steps or ingredients at the suggested or different parameter or concentration, may be suitably substituted. Though the ingredients or steps have been, in certain instances, described by reference to a particular function or result, it should be appreciated that such discussion is presented without intending to be bound by theory. In some instances, the ingredient or step will perform a different or an additional function or achieve a different result, or multiple other ingredients or steps may be substituted to perform such function or achieve such result. Thus, there is no intention to be bound to the breadth of any specific illustrative step, parameter, ingredient or concentration, where it is apparent that others may be advantageously be employed in addition to or as a substitute.

The present invention is further illustrated by particular reference to the following examples, it being understood that variations of the same may be made while still remaining within the scope of the invention. In the examples that follow, as well as in accordance with the preceding teachings (to which the discussion in this paragraph also applies), it is expected that the resulting cellular components will be capable of detection by an automated hematology analyzer. The resulting sizes of the cellular components may be substantially the same as the starting cells, larger or smaller. When different, the cellular components may range from about one half to about twice the size of the original cells. In certain instances, it is also possible that hemoglobin may be removed from within the cell, with removals (when occurring) ranging from about 0% to about 100% of the original hemoglobin (e.g., less than about 10%, less than about 20%, greater than about 70%, greater than about 85% or otherwise).

### Example 1

Chicken red blood cells are selected and run on an automated hematology analyzer. The cells are centrifuged for 10 minutes at 1000 rpm. The supernatant is aspirated and the cells are re-suspended in Solution D of Table 1 or 2. The cells are again centrifuged for 10 minutes at 1000 rpm followed by re-suspension of the cells in Solution D of Table 1 or 2. The centrifugation/re-suspension process is repeated for a third time. The red blood cell count is then adjusted to 200,000/µl using an automated hematology analyzer. A hypotonic fixative reagent is prepared, including a water solution containing 0.04% glutaraldehyde (percentage of stock glutaraldehyde which is 25%). The red blood cells are then added to the fixative reagent. The preparation is then incubated for 18 to 24 hours in a 40°C water bath. The cells are then re-suspended and centrifuged for 10 minutes at 1000 rpm. Supernatant is aspirated and the cells are re-suspended in approximately 300ml of a solution of phosphate buffered saline and a nonionic surfactant such as Triton X-705. The sample is centrifuged for 10 minutes at 1000 rpm. The supernatant is again aspirated and the cells are again washed with a solution of phosphate buffered saline and a nonionic surfactant such as Triton X-705. The centrifugation/re-suspension steps are repeated a third time. The cell count is then adjusted to approximately 50,000/µl using an automated hematology analyzer. A shrinking solution is then prepared by diluting isopropyl alcohol to 40% with water. 5ml of the shrinking solution is added to the cells which are then incubated for 18-24 hours at room temperature. The cells are then re-suspended and centrifuged for 10 minutes at 1000 rpm. The supernatant is aspirated and the cells are re-suspended in 300ml of a solution of phosphate buffered saline and 0.1% Triton X-705 surfactant. The cells are centrifuged (supernatant aspirated) and re-suspended in the phosphate buffered saline and 0.1% Triton X-705 surfactant solution twice more before final storage.

### Example 2

Turkey red blood cells are selected and run on an automated hematology analyzer. The cells are centrifuged for 10 minutes at 1000 rpm. The supernatant is aspirated and the cells are re-suspended in Solution D of Table 1 or 2. The cells are again centrifuged for 10 minutes at 1000 rpm followed by re-suspension of the cells in Solution D of Table 1 or 2. The centrifugation/re-suspension process is repeated for a third time. The red blood cell count is adjusted to 200,000/µl using an automated hematology analyzer. A hypotonic fixative solution is prepared, by diluting glutaraldehyde to 0.3% in water (percentage of stock glutaraldehyde which is 25%). The fix ratio is 1:100. 1ml of the cells is then added to 100ml the fixative solution. The preparation is then incubated for 18 to 24 hours in a 40°C water bath. The cells are then re-suspended and centrifuged for 10 minutes at 1000 rpm and the supernatant is aspirated. The cells are then re-suspended in a solution of phosphate buffered saline and 0.1% Triton X-705. The cells are centrifuged (supernatant aspirated) and re-suspended in a solution of phosphate buffered saline and 0.1% Triton X-705 twice more and the cell count is adjusted to approximately 100,000/µl on an automated hematology analyzer.

### Example 3

Human blood cells are contacted with 150ml of Solution E of Table 1 or 2 plus 0.11% formaldehyde for about 70 minutes at refrigerated temperature to stabilize the white blood cells. The cells are then centrifuged for 10 minutes at 1000 rpm and all but 100ml of the supernatant is removed. The red blood cells are lysed with ammonium chloride tris solution for 25-35 minutes at refrigerated temperature. All but 100ml of the supernatant is removed and the sample is again contacted with ammonium chloride tris solution for 60-70 minutes at refrigerated temperature. The sample is then washed with Solution F of Table 1 or 2 and the sample is centrifuged at a refrigerated temperature for 6 minutes at 600 rpm. The supernatant is carefully removed as the lymphocytes are located in the upper portion. The washing/centrifugation steps are repeated until the lymphocyte percentage is 10-15%. The white blood cells are then contacted with an expansion solution including Solution D of Table 1 or 2 for 60-70 minutes at refrigerated temperature. After expansion, the cells are contacted with 120ml of cold fix solution at room temperature for 48-120 hours. The cold fix solution contains 4% diazolidinyl urea, 20% formaldehyde, 0.5% Sorbitol, and 0.5% glutaraldehyde.

The cells are then washed with Solution B of Table 1 or 2 and re-suspended into Solution B of Table 1 or 2 with 5 g/l glycine and 1g/L L-ascorbic acid sodium salt and stored for 48-120 hours at refrigerated temperature. The supernatant is removed and the cells are contacted with Solution B of Table 1 or 2 and centrifuged for 10 minutes at 1000 rpm. The cells are then contacted with Solution C of Table 1 or 2 with 5% supertrate and 3mg% Proclin and stored.

### Example 4

An integrated control is prepared by mixing the cellular component of Example 2 with the cellular component of Example 3, such that the component of Example 2 includes lymphocyte analogs that replace the lymphocytes removed from the component of Example 3. The resulting mixture of Examples 2 and 3 is then combined with the component of Example 1. The resulting integrated control contains components capable of simulating both erythroblasts and white blood cells, including a three or five part differential of white blood cell subpopulations. When analyzed using an automated hematology analyzer, a readout may be obtained consistent with that of Fig. 2.

It will be appreciated that concentrates or dilutions of the amounts recited herein may be employed. In general, the relative proportions of the ingredients recited will remain the same. Thus, by way of example, if the teachings call for 30 parts by weight of a Component A, and 10 parts by weight of a Component B, the skilled artisan will recognize that such teachings also constitute a teaching of the use of Component A and Component B in a relative ratio of 3:1.

It will be appreciated that the above is by way of illustration only. Other ingredients may be employed in any of the compositions disclosed herein, as desired, to achieve the desired resulting characteristics. Examples of other ingredients that may be employed include antibiotics, anesthetics, antihistamines, preservatives, surfactants, antioxidants, unconjugated bile acids, mold inhibitors, nucleic acids, pH adjusters, osmolarity adjusters, or any combination thereof. Specific examples of ingredients that may be employed include one or more of sodium fluoride, a paraben (e.g., propyl), sulfasalazine, soybean protease inhibitor, sodium phosphate, potassium phosphate, sodium citrate, citric acid, sodium chloride, bovine serum albumin, sodium hydroxide, lipoprotein, Proclin, adenine, mannose, dextrose, lactose, penicillin, tetracycline, promethazine, a purine (e.g., adenine), inosine, kanamycin sulfate, cyclohexamide, deoxycholic acid, colistimethate sodium, trisodium citrate dehydrate, 5-Fluorouracil, or any combination thereof.

The explanations and illustrations presented herein are intended to acquaint others skilled in the art with the invention, its principles, and its practical application. Those skilled in the art may adapt and apply the invention in its numerous forms, as may be best suited to the requirements of a particular use. Accordingly, the specific embodiments of the present invention as set forth are not intended as being exhaustive or limiting of the invention. The scope of the invention should, therefore, be determined not with reference to the above description, but should instead be determined with reference to the appended claims, along with the full scope of equivalents to which such claims are entitled. The disclosures of all articles and references, including patent applications and publications, are incorporated by reference for all purposes. Other combinations are also possible as will be gleaned from the following claims, which are also hereby incorporated by reference into this written description.

## Claims

1. An integrated hematology control, comprising:
a simulated white blood cell subpopulation that includes:
a. a first portion of a simulated component derived from human white blood cells; and
b. a second portion of the simulated component derived from animal red blood cells;
wherein the first portion and second portion of the simulated component are processed for simulating a lymphocyte population of a human blood sample on an automated blood analyzer; and
wherein the control exhibits at least a white blood cell three part differential.

2. The control of claim 1 further comprising a component including a plurality of animal red blood cells processed for simulating an erythroblast population of a human blood sample on an automated blood analyzer.

3. The control of claim 2, wherein the animal red blood cells are derived from avian red blood cells.

4. The control of claim 2, wherein the animal red blood cells processed for simulating an erythroblast population of a human blood sample are derived from chicken red blood cells or alligator red blood cells.

5. The control of any of claims 1 through 4, wherein the second portion of the simulated component derived from animal red blood cell is derived from turkey red blood cells.

6. The control of any of claim 1 through 5, wherein the second portion of the simulated component derived from animal red blood cells makes up at least 50% of the lymphocytes in the lymphocyte population.

7. The control of any of claims 1 through 6, wherein the control exhibits a histogram that includes a valley between peaks associated with the erythroblast population and the lymphocyte population.

8. The control of any of claims 1 through 7, wherein the control exhibits a white blood cell five part differential.

9. A method of making the integrated hematology control of any of claims 2 through 7 comprising:
a. providing a component that simulates erythroblasts comprising the steps of:
i. providing a first portion of one or more animal red blood cells having a first volume;
ii. shrinking the first portion of one or more animal red blood cells to a volume at least 10% smaller than the first volume;
iii. contacting the first portion of one or more animal red blood cells with a surfactant;
iv. fixing the first portion of one or more animal red blood cells;
b. providing a component that simulates lymphocytes comprising the steps of:
i. i. providing a second portion of one or more animal red blood cells;
ii. contacting the second portion of one or more animal red blood cells with a surfactant;
ii. fixing the second portion of one or more animal red blood cells;
c. providing a component that simulates at least two subpopulations of white blood cells comprising the steps of:
i. providing a sample of whole human blood;
ii. lysing the red blood cells from the sample of whole human blood so that white blood cells remain;
iii. stabilizing the white blood cells;
iv. removing a portion of lymphocytes from the stabilized white blood cells;
d. replacing the removed portion of lymphocytes from the stabilized white blood cells with the component that simulates lymphocytes;
e. combining the component that simulates erythroblasts, and the component that simulates at least two subpopulations of white blood cells to form a control.

10. The method of claim 9, more than 50% of the lymphocytes from the stabilized white blood cells are removed and replaced with the component that simulates lymphocytes.

11. The method of claim 9, more than 70% of the lymphocytes from the stabilized white blood cells are removed and replaced with the component that simulates lymphocytes.

12. The method of any of claims 9 through 11, wherein the first portion of animal red blood cells, the second portion of animal red blood cells, or both are shrunk during processing.

13. The method of any of claims 9 through 12, wherein the first portion of animal red blood cells, the second portion of animal red blood cells, or both are contacted with a surfactant during processing.
